# DEMANDE DE BREVET EUROPEEN

(11) **EP 4 588 998 A1**
(43) Date de publication de la demande: **23.07.2025**
(21) Numéro de dépôt: 25152608.3
(22) Date de dépôt: 17.01.2025
(51) Int. Cl.: C12M 1/107, C02F 11/04, C12M 1/00

(54) **NOUVEAU DIGESTEUR ET INSTALLATION DE METHANISATION**

(30) Priorité: 19.01.2024 FR 2400535
(71) Demandeur: B.A.M Biogaz, 47110 Le Temple-sur-Lot (FR)
(72) Inventeur: FAURE, Jean-Marie, 78110 Le Vésinet (FR)
(74) Mandataire: Ipside

(57) **Abrégé**

La présente invention concerne un nouveau digesteur à enveloppe souple et une installation de méthanisation intégrant ledit digesteur de manière semi-enterrée. Le digesteur de l'invention est très avantageux en ce qu'il permet d'optimiser le nombre d'agitateurs à implémenter dans une telle installation grâce à la forme de sa partie inférieure destiné à recevoir le digestat. En particulière cette forme comporte des parois latérales inclinées et une section variable de forme polygonale présentant au moins 8 côtés, et de préférence de 8 à 12 côtés, et dans laquelle ladite section variable se rétrécie progressivement vers le fond du digesteur. L'invention concerne également une installation de méthanisation intégrant ledit digesteur sur un support au sol comportant une surface creuse s'adaptant à la forme de la partie inférieure du digesteur, et permettant d'intégrer de manière semi-enterrée ledit digesteur en limitant au maximum la profondeur nécessaire d'une telle excavation.

## Description

### DOMAINE TECHNIQUE DE L'INVENTION

La présente invention concerne le domaine de la méthanisation et de la production de biogaz à partir de matière organique. Plus précisément, l'invention concerne un nouveau digesteur et une installation de méthanisation intégrant ledit digesteur.

### ETAT DE LA TECHNIQUE

La méthanisation, également connue sous le nom de digestion anaérobie, est un processus biologique qui transforme les déchets organiques en biogaz, un mélange de méthane et de dioxyde de carbone. Le biogaz généré est une source d'énergie renouvelable qui peut être utilisée pour la production d'électricité, le chauffage et le transport. Le biogaz est donc une source d'énergie permettant de remplacer les combustibles fossiles et de ce fait, contribuant à la réduction des émissions de gaz à effet de serre. De plus, la capture du méthane dans le procédé de méthanisation permet de prévenir la libération de ce gaz dans l'atmosphère, émanant par exemple des déjections animales, et donc de prévenir l'effet du réchauffement climatique. La méthanisation produit également un digestat riche en nutriments qui peut être utilisé comme engrais pour les cultures.

Les exploitations agricoles ont donc un grand intérêt pour avoir leur propre installation de méthanisation. Or, cette installation n'est pas toujours viable en fonction de la taille de l'exploitation agricole et l'investissement nécessaire pour construire et/ou opérer une telle installation. Il est donc souhaitable d'avoir des installations accessibles en termes d'investissement pour les petites et les moyennes exploitations agricoles.

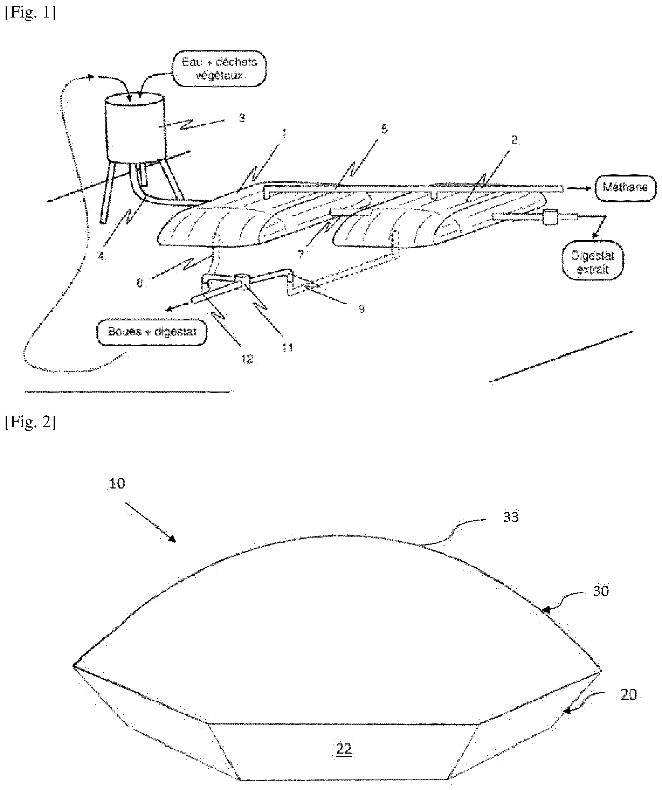

Le brevet EP2 703 364 B1 au nom d'ARCBIOGAZ décrit une installation répondant à ces besoins. Comme illustré sur la [Fig.1], cette installation met en oeuvre deux digesteurs, un premier digesteur 1, et un post-digesteur 2 reliés entre eux, et présentant respectivement une forme rectangulaire en matière souple, tel que du PVC. Les digesteurs dits « en poche souple » sont posés directement sur le sol, ce dernier étant excavé de sorte à former un support pour les digesteurs. En particulier, une partie basse du digesteur contenant la matière liquide et/ou solide repose sur la partie basse de l'excavation, et la partie haute du digesteur contenant le biogaz est en surélévation par rapport au sol. L'installation comporte également une pré-cuve de mélange 3 en surélévation et permettant d'homogénéiser les déchets agricoles avec de l'eau avant son introduction dans le premier digesteur 1 au moyen d'une canalisation 4. La digestion de déchets est effectuée en priorité dans le premier digesteur 1, et le digestat résultant est ensuite transféré au moyen d'une canalisation de raccord 7 au post-digesteur pour finaliser la méthanisation de la matière organique restante avant extraction. Le biogaz produit est extrait à partir du haut des digesteurs 1, et 2 au moyen d'une canalisation 15. De manière particulière, les digesteurs 1, 2 sont pourvus d'une sortie inférieure reliée à une pompe de recirculation 11, de sorte que la boue et le sable accumulé au fond de digesteurs soient extraits et transférés à la pré-cuve au moyen d'un système de canalisations 8, 9, 12 puis réinjectés dans le premier digesteur. Cette recirculation de boues permet d'améliorer l'efficacité de la méthanisation et de réduire au maximum les opérations de maintenance du système.

L'installation de méthanisation décrite ci-dessus est très avantageuse en ce qu'elle réduit significativement les coûts d'investissement vis-à-vis d'une installation de méthanisation classique et ne requière pas d'infrastructure en béton. D'autre part, cette installation nécessite d'environ quatre agitateurs positionnés en périphérie du digesteur pour assurer le bon fonctionnement du système. Les coûts de l'énergie électrique ayant fortement augmenté, il est maintenant souhaitable d'optimiser ce système pour diminuer au maximum la consommation électrique des agitateurs et augmenter l'efficacité du système. La présente invention permet de répondre à ce besoin.

### Exposé de l'invention

La présente invention concerne un nouveau digesteur à enveloppe souple et une installation de méthanisation intégrant ledit digesteur de manière semi-enterrée.

Plus particulièrement, l'invention porte sur un digesteur présentant un corps en matériau étanche souple comportant une partie inférieure définissant un récipient pour recevoir un digestat, et une partie supérieure définissant un toit gonflable pourvu d'une sortie de gaz, caractérisé en ce que la partie inférieure du corps du digesteur comporte :
- des parois latérales inclinées et une section variable de forme polygonale présentant au moins 8 côtés, et de préférence de 8 à 12 côtés,
- et dans laquelle ladite section variable se rétrécie progressivement vers un fond du digesteur.

Avantageusement, la section variable de forme polygonale de la partie inférieure du digesteur permet d'optimiser l'agitation du digestat, tout en optimisant la surface au sol nécessaire pour supporter ledit digesteur. En particulier, cette forme permet de limiter la profondeur d'excavation nécessaire pour intégrer de manière semi-enterrée ledit digesteur au sol et d'implémenter 1 agitateur en périphérie du digesteur tous les environ 1000 m³.

Afin d'augmenter d'avantage l'efficacité d'agitation, le fond du digesteur est convexe ou présente une forme sensiblement conique ou pyramidale, et un centre axial du fond du digesteur correspond à la partie la plus basse du digesteur. De préférence, le fond du digesteur présente une inclinaison inférieure à une inclinaison des parois latérales de la partie inférieure du digesteur.

Selon un mode de réalisation, un premier angle d'inclinaison des parois latérales de la partie inférieure du digesteur est compris entre 30° et 60°, et deuxième angle inclinaison du fond du digesteur est compris entre 10° et 30°.

En ce qui concerne la partie supérieure du digesteur, celle-ci présente une forme de dôme en position étendue et lorsque le gaz de méthanisation s'accumule à son intérieur.

Le digesteur étant réalisée en matériau souple et flexible, la partie supérieure du digesteur perd sa forme hémisphérique en absence d'accumulation de gaz. La partie supérieure du digesteur est donc pourvue de préférence d'une bouée sur sa surface interne, cette bouée étant destinée à maintenir à flotte des capteurs positionnés sur la partie supérieure du digesteur (lorsque celle-ci est dégonflée).

La présente invention concerne également une installation de méthanisation comportant un digesteur selon l'invention, et comportant également un support au sol sur lequel est posé ledit digesteur. Cette installation est particulière en ce que :

- ledit support comporte une surface creuse adoptant sensiblement la forme de la partie inférieure dudit digesteur, et est formé par une excavation au sol et une digue entourant ladite excavation.

Dans cette installation, l'excavation comporte des parois surélevées par ladite digue entourant l'excavation et de sorte que lorsque le digesteur est posé sur ledit support, les parois latérales de la partie inférieure du digesteur sont partiellement enterrées et partiellement surélevées par rapport au sol.

Bien entendu l'installation de méthanisation ci-dessus est à implémenter en combinaison avec tous les équipements et infrastructure nécessaire pour son fonctionnement.

Notamment, l'installation comporte au moins un agitateur au niveau des parois du support du digesteur surélevées par ladite digue, ledit au moins un agitateur étant intégrée dans une fosse bétonnée dans ladite digue, et comportant de préférence une porte amovible de maintenance sur un côté de la fosse en contact avec ledit digesteur.

Selon un mode de réalisation, l'installation comporte une pré cuve surélevée reliée au digesteur pour alimenter en matière organique ladite installation, et un post digesteur alimenté par le digesteur et étant posé au sol sur un deuxième support. Dans cette installation, le digesteur et le post-digesteur sont pourvus d'une sortie inférieure reliée à la pré cuve au moyen d'un système de recirculation comportant une série de canalisations et une pompe de recirculation, ledit système de recirculation étant configurée pour extraire l'accumulation de boue et de sable au fond du digesteur et du post digesteur, et pour la transférer à la pré-cuve pour sa réinjection dans le digesteur.

Bien entendu, les parties supérieures du premier et du deuxième digesteur sont reliées à un système d'extraction de gaz.

### BRÈVE DESCRIPTION DES FIGURES

D'autres avantages, buts et caractéristiques particulières de la présente invention ressortiront de la description non limitative qui suit d'au moins un mode de réalisation particulier des dispositifs et procédés objets de la présente invention, en regard des dessins annexés, dans lesquels :
La [Fig.1] représente une installation de méthanisation aux digesteurs « en poche souple » selon l'état de la technique.
La [Fig.2] représente schématiquement une vue en perspective d'un digesteur en poche souple selon l'invention.
La [Fig.3] représente le digesteur de la [Fig.2] vue avec une transparence au niveau du toit du digesteur.
La [Fig.4] représente un deuxième mode de réalisation du fond du digesteur de la [Fig.2] représenté avec une transparence au niveau de son toit.
La [Fig.5] représente schématiquement une section verticale d'un digesteur de l'invention.
La [Fig.6] représente schématiquement un support construit par terrassement du sol et destiné à recevoir un digesteur selon l'invention.
La [Fig.7] représente schématiquement une coupe verticale d'une installation comportant un support au sol recevant un digesteur selon l'invention.

### DESCRIPTION DETAILLEE DE L'INVENTION

La présente invention concerne un digesteur et une installation de méthanisation optimisée pour être déployée à des coûts accessibles pour des petites et des moyennes exploitations agricoles. En particulier, l'invention propose une nouvelle forme de digesteur permettant de réduire les coûts d'opération en électricité d'une installation à digesteurs en enveloppe souple, et notamment concernant l'énergie consommé au niveau de l'agitation.

Les figures 1 à 6 illustrent schématiquement le digesteur de l'invention. Ces figures ne sont pas à l'échelle et sont données à titre illustratif et non limitatif de l'invention.

Le digesteur 10 de l'invention est particulier en ce qu'il est fabriqué en matériau souple, tel que du PVC ou tout autre matière plastique étanche capable de former une enveloppe flexible, dite aussi « poche souple » et agissant comme cuve de fermentation. Pour les besoins de la description, le digesteur sera décrit par rapport à une position d'utilisation et lorsqu'il est installé au sol.

Le digesteur de l'invention est formé par une partie inférieure 20 et une partie supérieure 30. La partie inférieure est destinée à être posé sur le sol semi-enterrée et à recevoir le digestat, notamment un mélange des déchets agricoles et de l'eau. La partie supérieure 30 définit un toit gonflable relié à la partie inférieure, et est destiné à contenir le gaz produit par la réaction de méthanisation.

La partie inférieure 20 du corps du digesteur comporte des parois latérales 22 inclinées vers un centre axial et vers un fond 24 du digesteur. De manière caractéristique, la partie inférieure 20 du digesteur présente une section variable de forme polygonale présentant au moins huit côtés, et pouvant aller de huit côtés à douce côtés. Tel qu'illustré sur les figures, la section variable de la partie inférieure du digesteur se rétrécie graduellement jusqu'au fond du digesteur. Le digesteur comporte ainsi une section médiane 21, de forme polygonale divisant la partie supérieure et la partie inférieure du digesteur. La partie supérieure 30 s'étend vers le haut comme un dôme (sous effet de l'accumulation de gaz), alors que la partie inférieure à une forme général de polyèdre. Nous pouvons considérer que ce polyèdre a comme bases la section médiane 21 du digesteur et une section inférieure 23 présentant la même forme polygonale (à section rétrécie) et se situant au niveau du fond 24 du digesteur.

Avantageusement, cette section variable à forme polygonale permet au digesteur 10 de l'invention d'avoir une efficacité d'agitation proche de celle d'un digesteur rond en béton. Cependant, au contraste d'un digesteur rond en béton, le digesteur de l'invention n'a pas besoin d'infrastructure coûteuse ou d'excavation profonde nécessitant d'un type de sol adapté à une telle excavation. Au contraire, dans la présente invention la forme du digesteur 10 permet d'optimiser la surface au sol et la profondeur de l'excavation nécessaire pour supporter le digesteur. La nappe phréatique ne sera pas non plus atteinte vis-à-vis de la profondeur d'excavation nécessaire, et qui sera d'environ 2m à 3,5m maximum. De plus, cette forme est également propice à accélérer la fermentation anaérobique du digestat.

De préférence, le fond 24 de la partie inférieure 20 est convexe ou présente une forme légèrement conique ou pyramidale dont le sommet correspond à la partie la plus basse du digesteur. Le fond 24 du digesteur intègre également une sortie pour évacuer la boue et le sable accumulés au fond du digesteur (non illustrée).

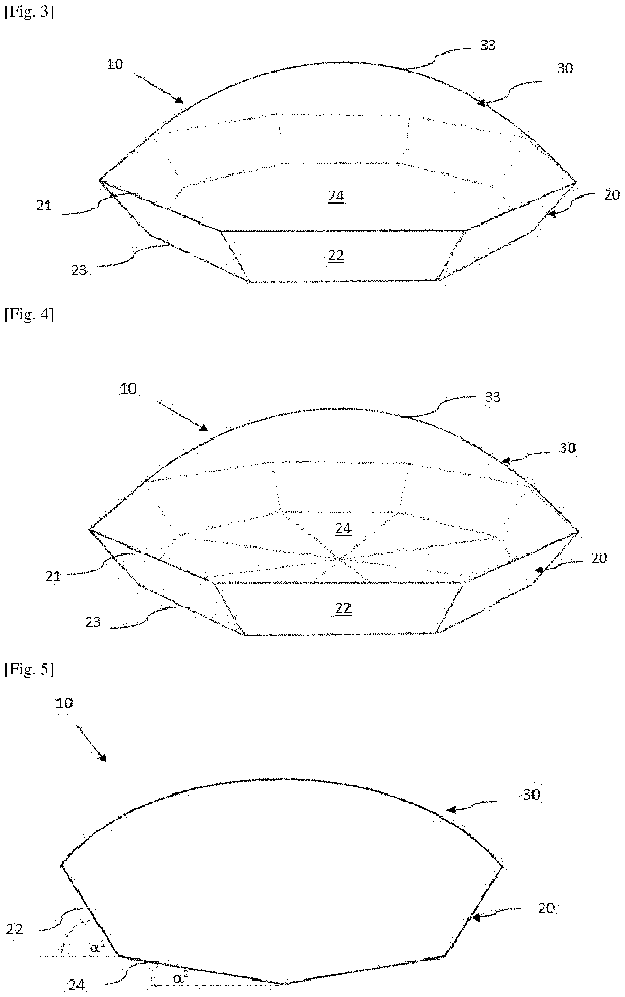

Dans un mode de réalisation préféré (illustré à la [Fig.4]), le fond 24 de la partie inférieure 20 présente une forme pyramidale ayant pour base la section inférieure 23 du corps du digesteur. Le fond 24 du digesteur, et plus particulièrement des parois du fond du digesteur s'étendent donc en continuité des parois latérales 22 du digesteur, mais selon une deuxième inclinaison. De préférence, l'inclinaison du fond du digesteur est inférieure à celle des parois latérales de la partie inférieure du digesteur. La [Fig.5] illustre une coupe du digesteur selon un plan vertical, et montrant la première inclinaison des parois latérales du digesteur, et la deuxième inclinaison du fond du digesteur. Ces inclinaisons sont choisies d'une part pour optimiser le volume au sol et l'agitation. D'autre part, ces inclinaisons sont choisies pour avoir un ratio remblais/ déblais proche de 1. Il sera donc possible de limiter au mieux les besoins d'apport de matériaux ou d'évacuation de remblais. Par exemple, un angle α¹ d'inclinaison des parois latérales 22 est compris entre 30° et 60°, et un angle α² d'inclinaison du fond du digesteur est compris entre 10° et 30°. Selon un mode de réalisation, le premier angle α ¹est de 45°, et le deuxième angle α² est de 15°.

Cette forme permettra d'optimiser d'avantage l'agitation du digestat, ainsi que de faciliter les travaux de terrassement nécessaires à l'installation du digesteur. Cette forme permet également de réduire le nombre de soudures nécessaires pour fabriquer le digesteur.

La présente invention concerne également une installation de méthanisation comportant au moins un support au sol et un digesteur selon l'invention. En particulier, le support au sol est défini par un terrassement du sol adoptant la forme de la partie inférieure du digesteur et permettant donc de maintenir la conformation de la forme caractéristique de la partie inférieure du digesteur.

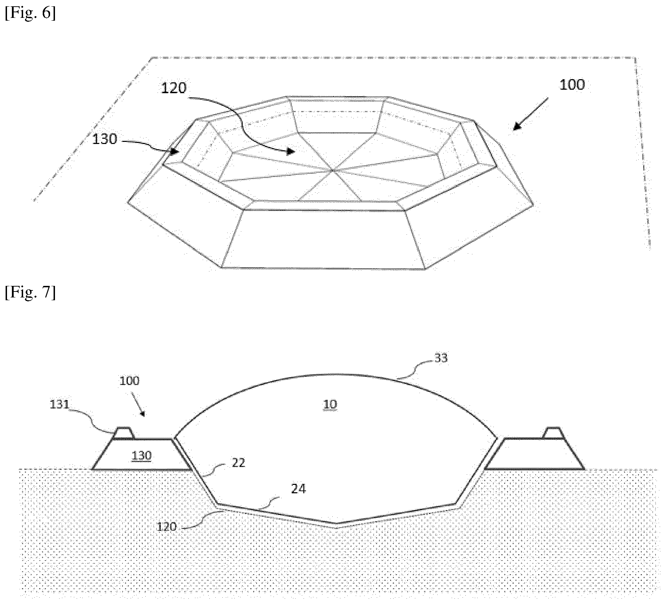

Comme illustré sur la [Fig.6], ce support est formé par une excavation 120 au sol et par une digue 130 entourant ladite excavation. Afin de se conformer à la forme de la partie inférieure du digesteur, l'excavation est pourvue également des parois latérales inclinées, ces dernières étant surélevées par rapport au sol, au moyen de ladite digue. Il est ainsi obtenu un support avec des parois latérales partiellement enterrées et partiellement surélevées par rapport au niveau du sol. Sur la [Fig.6], les lignes pointillées signalent le niveau du sol au niveau des parois latérales internes du support, et séparant l'excavation 120 de la digue 130 entourant ladite excavation. Bien entendu, une section interne de l'excavation 120 et de la digue 130 présente aussi une forme polygonale à section variable, tel que la forme octogonale illustrée sur la [Fig.6].

Comme illustré sur la [Fig.7] selon une coupe verticale de l'installation, la digue 130 a pour sa part un corps de prisme trapézoïdale permettant de former une structure de contention de parois latérales en surélévation du support. La forme trapézoïdale de cette digue est avantageuse en ce qu'elle permet de former une banquette sur une surface supérieure de la digue et tout autour du digesteur, facilitant ainsi les interventions du personnelle et l'intégration d'équipements. La digue peut être également pourvue à titre de sécurité d'un débord 131 sur sa surface supérieure permettant de contenir un éventuel débordement de liquide ou du digesteur rempli, en cas de fuite sur ce dernier. En fonctionnement nominal, le liquide sera affleurant au niveau de la digue, supprimant ce risque.

Selon un mode de réalisation, cette digue sera construite à partir du remblai des travaux de terrassement, et aura une largeur d'environ 2 à 4 mètres. Bien entendu la digue sera stabilisée pour éviter son déplacement. Selon un mode de réalisation, des butées étanches sont intégrées autour de la digue tous les 1 à 2 mètres. La forme de la digue n'est pas limitative et pourra être adaptée au cas par cas de lorsque sa forme permette de prolonger et stabiliser les parois de l'excavation selon la forme prismatique de la partie inférieure du digesteur. La digue servira également de support d'intégration à ou aux agitateurs, ce ou ces derniers étant intégrées en périphérie du digesteur dans une fosse bétonnée sur 3 côtés, la paroi en contact avec le digesteur étant métallique et amovible pour maintenance. L'agitateur est par exemple un agitateur externe pour cuve de béton, s'étendant vers le digestat à partir de la paroi interne de la digue en contact avec le digestat. Cette installation de l'agitateur permet de cibler un niveau de liquide dans le digesteur au niveau du haut de la digue, ce qui réduit les risques et les contraintes sur la partie haute du digesteur.

Les dimensions du digesteur et de son support seront choisies en fonction du volume de digestat à traiter. L'installation de l'invention est recommandée pour traiter de volumes de digestat allant d'environ 500m³ à 5000m³. De manière générale, la partie supérieure du digesteur sera dimensionnée avec une hauteur équivalente à ½ à ¼ du diamètre du digesteur, ce diamètre étant en moyenne de 13 à 37 mètres.

L'installation de méthanisation de l'invention est très avantageuse car elle peut être déployée avec un coût d'investissement nettement inférieur à celui des installations classiques. De plus, cette installation peut être implémentée sur presque tout type de terrain, car la profondeur maximale d'excavation sera d'environ 3,5 mètres maximum. La forme particulière du digesteur de l'invention permet de déployer l'installation décrite dans le brevet EP2 703 364 B1 mais avec un nombre réduit d'agitateurs. En effet, l'invention permet de passer d'un agitateur tous les 120m³ à un agitateur pour 1000m³ à 1400m³. Les coûts d'opération, et notamment les coûts d'électricité d'une telle installation sont ainsi réduits de manière très importante, et permettant aux exploitations de mieux rentabiliser leur installation de méthanisation.

## Revendications

1. Digesteur (10) présentant un corps en matériau étanche souple comportant une partie inférieure (20) définissant un récipient pour recevoir un digestat, et une partie supérieure (30) définissant un toit gonflable pourvu d'une sortie de gaz, **caractérisé en ce que** la partie inférieure (20) du corps du digesteur comporte :
- des parois latérales (22) inclinées et une section variable de forme polygonale présentant au moins 8 côtés, et de préférence de 8 à 12 côtés, et
- et dans lequel ladite section variable se rétrécie progressivement vers un fond (24) du digesteur.

2. Digesteur selon la revendication 1, dans lequel le fond (24) du digesteur (10) est convexe ou présente une forme sensiblement conique ou pyramidale, et dans lequel un centre axial du fond (24) du digesteur correspond à la partie la plus basse du digesteur.

3. Digesteurs selon la revendication 2, dans lequel le fond (24) du digesteur présente une inclinaison inférieure à une inclinaison des parois latérales (22) de la partie inférieure (20) du digesteur.

4. Digesteur selon la revendication 3, dans lequel un premier angle (α1) d'inclinaison des parois latérales (22) de la partie inférieure (20) du digesteur est comprise entre 30° et 60°, et un deuxième angle (α2) inclinaison du fond (24) du digesteur est compris entre 10° et 30°.

5. Digesteur selon l'une des revendications 1 à 4, dans lequel la partie supérieure (30) du digesteur présente une forme de dôme en position étendue et lorsque le gaz de méthanisation s'accumule à son intérieur.

6. Digesteur selon l'une des revendications précédentes, dans lequel la partie supérieure (30) du digesteur est pourvue d'une bouée sur sa surface interne, cette bouée étant destinée à maintenir à flotte des capteurs positionnés sur la partie supérieure du digesteur.

7. Installation de méthanisation comportant un digesteur (10) selon l'une des revendications 1 à 6, et comportant également un support (100) au sol sur lequel est posé ledit digesteur (10), **caractérisé en ce que** :
- ledit support (100) comporte une surface creuse adoptant sensiblement la forme de la partie inférieure (20) dudit digesteur (10), et est formé par une excavation (120) au sol et une digue (130) entourant ladite excavation (120).

8. Installation selon la revendication 7, dans laquelle l'excavation comporte des parois surélevées par ladite digue (130) entourant l'excavation et de sorte que lorsque le digesteur (10) est posé sur ledit support (100), les parois latérales (22) de la partie inférieure (20) du digesteur sont partiellement enterrées et partiellement surélevées par rapport au sol.

9. Installation selon la revendication 7 ou 8, comportant une pré cuve surélevée reliée au digesteur pour alimenter en matière organique ladite installation, et un post digesteur alimenté par le digesteur et étant posé au sol sur un deuxième support, et dans laquelle installation le digesteur et le post-digesteur sont pourvus d'une sortie inférieure reliée à la pré cuve au moyen d'un système de recirculation comportant une série de canalisations et une pompe de recirculation, ledit système de recirculation étant configurée pour extraire l'accumulation de boue et de sable au fond du digesteur et du post digesteur, et pour la transférer à la pré cuve pour sa réinjection dans le digesteur.

10. Installation selon la revendication 9, dans lequel les parties supérieures du premier et du deuxième digesteur sont reliées à un système d'extraction de gaz.

11. Installation selon l'une de revendications 8 à 10, comportant au moins un agitateur au niveau des parois du support (100) du digesteur (10) surélevées par ladite digue (130), ledit au moins un agitateur étant intégrée dans une fosse bétonnée dans ladite digue, et comportant de préférence une porte amovible de maintenance sur un côté de la fosse en contact avec ledit digesteur.
